# EUROPÄISCHE PATENTSCHRIFT

(11) **EP 1 886 690 B1**
(45) Veröffentlichungstag und Bekanntmachung des Hinweises auf die Patenterteilung: **28.09.2016**
(21) Anmeldenummer: 07002516.8
(22) Anmeldetag: 06.02.2007
(51) Int. Cl.: A61K 36/185, A61K 31/352, A61K 38/47, A61K 31/4415, A61P 1/00

(54) **Pharmazeutische Zusammensetzung zur Anwendung bei Nahrungsmittelunverträglichkeiten**
Pharmaceutical composition for the treatment of food intolerance
Composition pharmaceutique pour le traitement de l'intolérance alimentaire

(30) Priorität: 10.08.2006 DE 102006037612
(43) Veröffentlichungstag der Anmeldung: 13.02.2008
(73) Patentinhaber: Maria Clementine Martin Klosterfrau Vertriebsgesellschaft mbH, 50670 Köln (DE)
(72) Erfinder: Vestweber, Anna-Maria, Dr. med., 51399 Burscheid (DE); Bökemeier, Rolf, 41540 Dormagen (DE)
(74) Vertreter: Von Rohr Patentanwälte Partnerschaft mbB

(56) Entgegenhaltungen:
- DATABASE IMSPRODUCT [Online] Drug Launches 28. Juni 2006 (2006-06-28), XP002524182 gefunden im STN Database accession no. 2006:9137
- "Hilfe bei Symptomen an Magen und Darm" AERZTE ZEITUNG, AERZTE ZEITUNG, NEU ISENBURG, DE, Bd. 25, Nr. 99, 31. Mai 2006 (2006-05-31), Seite 10, XP009115413 ISSN: 0175-5811
- ANONYM: "Allergolact (Syxyl GmbH & Co.KG" LIBASE INFOTHEK, [Online] XP002524181 Gefunden im Internet: URL:http://www.libase.de/Praeparat_Allergo lact_Syxyl_GmbH_und_CoKG_T14271.html> [gefunden am 2009-04-17]
- FRIESE ET AL: "Homöopatische Behandlung von Nahrungsmittelallergien" NATURARZT, XX, DE, Bd. 4, 1. Januar 1998 (1998-01-01), Seite 34, XP009114876 ISSN: 0028-081X

## Beschreibung

Die vorliegende Erfindung betrifft eine Zusammensetzung, insbesondere pharmazeutische Zusammensetzung bzw. Zubereitung, insbesondere zur Anwendung bei Nahrungsmittelunverträglichkeiten.

Insbesondere betrifft die vorliegende Erfindung eine Zusammensetzung, insbesondere in Form einer pharmazeutischen Zubereitung, welche sich für die prophylaktische und/oder kurative Behandlung von Nahrungsmittelunverträglichkeiten mit Magen/Darm-Beschwerden eignet.

Des weiteren betrifft die vorliegende Erfindung die Verwendung dieser Zusammensetzung zur prophylaktischen und/oder kurativen, insbesondere symptomatischen prophylaktischen und/oder kurativen Behandlung bzw. zur Herstellung eines Arzneimittels zur prophylaktischen und/oder kurativen, insbesondere symptomatischen prophylaktischen und/oder kurativen Behandlung von Nahrungsmittelunverträglichkeiten (d. h. nichtallergenen Nahrungsmittelintoleranzen) mit Magen/ Darm-Beschwerden, insbesondere Kohlenhydratmalabsorption, wie Monosaccharidmalabsorption und Disaccharidmalabsorption, so z. B. Laktoseintoleranz (Laktoseunverträglichkeit, Alaktasie), Saccharoseintoleranz und Maltosintoleranz.

Der Begriff der Nahrungsmittelunverträglichkeiten - d. h. Nahrungsmittelintoleranzen, welche nicht allergisch bedingt sind - bezeichnet die Unfähigkeit, ein bestimmtes Nahrungsmittel oder einen bestimmten Nahrungsmittelbestandteil zu verdauen. Im Fall einer Nahrungsmittelintoleranz hat der Körper die Fähigkeit verloren, einen bestimmten Stoff zu verdauen, oder diese Fähigkeit nie besessen. Im Gegensatz zu einer Nahrungsmittelallergie treten bei der Nahrungsmittelunverträglichkeit bzw. Nahrungsmittelintoleranz keine Immunreaktionen auf, da keine Antikörper gebildet werden. Dennoch können bisweilen die Symptome einer Nahrungsmittelunverträglichkeit bzw. -intoleranz denen einer Allergie stark ähneln, so daß man in solchen Fällen bisweilen auch von sogenannten Pseudoallergien spricht. Ursache einer solchen Nahrungsmittelunverträglichkeit bzw. -intoleranz können beispielsweise Enzymdefekte oder ein Enzymmangel sein. Darum können bestimmte Nahrungsmittel nicht verdaut werden. Ein klassisches Beispiel hierfür ist die sogenannte Laktoseintoleranz, eine Krankheit, bei welcher es aufgrund eines Mangels an dem milchzuckerspaltenden Enzym Lactase insbesondere zu Durchfällen und Blähungen kommt. Nahrungsmittelunverträglichkeiten können sowohl erworben als auch angeboren auftreten. In Abhängigkeit von der Schwere der Nahrungsmittelintoleranz können die betroffenen Menschen unter Umständen weiterhin geringe Mengen an diesen Nahrungsmitteln zu sich nehmen, ohne daß sich bei ihnen Symptome einstellen; bei anderen Erkrankten dagegen ist die Nahrungsmittelaufnahme der betreffenden Nahrungsmittel überhaupt nicht mehr möglich.

Die häufigste Form der Nahrungsmittelintoleranz ist die sogenannte Kohlenhydratmalabsorption. Unter einer Malabsorption versteht man die ungenügende Aufnahme von Nahrungsbestandteilen aus dem Verdauungstrakt, unter anderem dem Dünndarm, und zwar infolge einer Störung der Verdauung (Maldigestion) oder im engeren Sinne der Resorption. Unter einer Kohlenhydratmalabsorption versteht man die angeborene oder erworbene Störung des Abbaus oder der Resorption verschiedener Kohlenhydrate. Klinische Leitsymptome sind Durchfälle, die osmotisch bedingt bzw. Folge der Vergärung der Kohlenhydrate durch Darmbakterien sind. Erworbene Defekte sind oft die Folge einer Atrophie der Mukosazellen mit Aktivitätsverlust der dort lokalisierten Disaccharidasen, wobei die Lactase wegen ihrer geringen Aktivität meist am stärksten betroffen ist.

Man unterscheidet verschiedene Formen der Kohlenhydratmalabsorption, zum einen die Monosaccharidmalabsorption (z. B. Glukose-Galaktose-Malabsorption) und die Disaccharidmalabsorption (z. B. Lactoseintoleranz bzw. -unverträglichkeit, auch als Alaktasie bezeichnet, Saccharoseintoleranz, Saccharose-Isomaltose-Malabsorption und Maltoseintoleranz).

Am häufigsten tritt dabei die zuvor bereits erwähnte Lactoseintoleranz auf, eine Milchzuckerunverträglichkeit, bei der ein Bestandteil der Milch, nämlich die Lactose (= Milchzucker), Unverträglichkeiten hervorruft, wobei der Schweregrad der Lactoseintoleranz individuell sehr unterschiedlich ist und unter anderem davon abhängt, ob die Lactase (d. h. das milchzuckerspaltende Enzym) vollständig fehlt oder ob noch eine Restfunktion vorhanden ist. Ursache für die Milchzuckerunverträglichkeit ist das Fehlen bzw. die unzureichende Produktion des Verdauungsenzyms Lactase, welches notwendig ist, um den Milchzucker in seine Einzelbestandteile (= Glucose und Galaktose) zu spalten, welche dann in das Blut aufgenommen werden können. Bei gesunden Menschen kommt das Verdauungsenzym Lactase in der Dünndarmschleimhaut vor. Wird der Milchzucker aber nicht gespalten und gelangen größere Mengen in untere, mit Bakterien besiedelte Darmabschnitte, dient der Milchzucker den Bakterien als Nährsubstrat. Es entstehen große Mengen an Gasen und organischen Säuren, welche ein Einströmen von Wasser in den Darm sowie vermehrte Darmbewegungen bewirken. Die Folgen bzw. Symptome einer Milchzuckerunverträglichkeit sind unter anderem Bauchschmerzen und Koliken, Völlegefühl, Blähungen, Übelkeit und Durchfall.

Bei einem Lactasemangel wird zwischen primärem und sekundärem Lactasemangel unterschieden: Bei einem primären Lactasemangel werden abhängig vom Zeitpunkt der Manifestation wiederum zwei Formen unterschieden, nämlich der neonatale Lactasemangel, eine erbliche Stoffwechselkrankheit, welche sehr selten auftritt, und der physiologische Lactasemangel, der nach dem Abstillen beginnt, wobei die Lactaseaktivität stark zurückgeht. Letztere Form ist weltweit verbreitet. Beim sekundären Lactasemangel dagegen ist der Lactasemangel Begleiterscheinung einer anderen Krankheit, wie z. B. Zöliakie oder Morbus Crohn; wenn die auslösende Erkrankung abgeheilt ist, kann auch wieder ausreichend Lactase gebildet werden.

Nahrungsmittelintoleranzen treten regional stark unterschiedlich auf: Die Mehrzahl der Weltbevölkerung (ca. 90 %) kann Milchzucker nach dem Säuglingsalter nicht mehr vollständig verwerten. In asiatischen Ländern beispielsweise fehlt den meisten Menschen das Enzym zur Spaltung des Milchzuckers. Daher findet man in diesen Regionen auch keine Milch- oder Käseprodukte auf dem Speiseplan. In Mitteleuropa leiden ca. 10 bis 20 % der Durchschnittsbevölkerung an einer Lactaseintoleranz.

Bei einem Lactasemangel besteht die Therapie in der Reduzierung bzw. Meidung des Verzehrs von Milch und Milchprodukten und anderen lactasehaltigen Produkten. Da diese Lebensmittel aber Hauptlieferanten des Mineralstoffs Calciums sind, der unter anderem für die Stabilität des Knochengerüstes mitverantwortlich ist, besteht bei Menschen mit Lactasemangel die Gefahr einer Osteoporose.

Aus dem Stand der Technik sind grundsätzlich Produkte bzw. Präparate bekannt, welche Okoubaka und Quercetin enthalten und zur Behandlung von Nahrungsmittelunverträglichkeiten eingesetzt werden. Darüber hinaus existieren Präparate, welche getrocknete Okoubakarinde, Tilactase, Acerolafruchtpulver, Pyridoxinhydrochlorid und Quercetin enthalten und ebenfalls gegen Magen/Darm-Beschwerden wegen Nahrungsmittelunverträglichkeiten eingesetzt werden.

Es ist daher die Aufgabe der vorliegenden Erfindung, eine Zusammensetzung bzw. ein Mittel bereitzustellen, welche bzw. welches sich insbesondere für die Behandlung von Nahrungsmittelunverträglichkeiten bzw. Nahrungsmittelintoleranzen, insbesondere Nahrungsmittelunverträglichkeiten mit Magen/Darm-Beschwerden, eignet.

Die Anmelderin hat nunmehr überraschenderweise herausgefunden, daß die zuvor geschilderte Aufgabe dadurch gelöst werden kann, daß man Okoubaka (d. h. die Inhaltsstoffe aus der Rinde des im westafrikanischen Regenwald beheimateten Baumes Okoubaka aubrevillei) und 3,3',4',5,7-Pentahydroxyflavon zusammen mit mindestens einer Galactosidase und mit Pyridoxinhydrochlorid (Vitamin B6) kombiniert bzw. appliziert bzw. formuliert.

Gegenstand der vorliegenden Erfindung - gemäß einem **ersten** Aspekt der vorliegenden Erfindung - ist somit eine Zusammensetzung in Form einer pharmazeutischen Zubereitung zur Anwendung bei der prophylaktischen und/oder kurativen Behandlung von Nahrungsmittelunverträglichkeiten mit Magen/Darm-Beschwerden, wobei die Zusammensetzung - in Kombination und in jeweils pharmazeutisch wirksamen Mengen - enthält:
(a) Okoubaka, wobei die Zusammensetzung Okoubaka in Form eines Extraktes in homöopathischer Form und Menge von Dil. D4 in Mengen von 12 bis 18 Ges.-%, bezogen auf die Zusammensetzung, enthält;
(b) 3,3',4',5,7-Pentahydroxyflavon, wobei die Zusammensetzung 3,3',4',5,7-Pentahydroxyflavon in Mengen von 5 bis 10 Gew.-%, bezogen auf die Zusammensetzung, enthält;
(c) β-D-Galactosid-Galactohydrolase (Lactase), wobei die Zusammensetzung β-D-Galactosid-Galactohydrolase (Lactase) in Mengen von 0,5 bis 5 Ges.-%, bezogen auf die Zusammensetzung, enthält;
(d) Pyridoxinhydrochlorid (Vitamin B6), wobei die Zusammensetzung Pyridoxinhydrochlorid (Vitamin B6) in Mengen von 0,1 bis 1 Gew.-%, bezogen auf die Zusammensetzung, enthält; und
(e) Ascorbinsäure (Vitamin C) in Form von Acerolapulver, wobei die Zusammensetzung Acerolapulver in Mengen von 20 bis 30 Gew.-%, bezogen auf die Zusammensetzung, enthält;
wobei die Zusammensetzung außerdem einen pharmazeutischen Träger enthält und
wobei die Zusammensetzung in peroral verabreichbarer Applikationsform in fester Dosierungsform als Filmtablette oder Kapsel mit magensaftresistentem und/oder dünndarmlöslichem Überzug oder Umhüllung vorliegt.

Der Begriff "pharmazeutische Zusammensetzung" bzw. "pharmazeutische Zubereitung", wie er im Rahmen der vorliegenden Erfindung verwendet wird, ist sehr breit zu verstehen und umfaßt nicht nur pharmazeutische Präparate bzw. Pharmazeutika und Arzneimittel als solche, sondern auch sogenannte Medizinprodukte sowie Homöopathika und darüber hinaus auch Nahrungsergänzungsmittel, Nahrungsmittelzusätze und Diätetika.

Die Anmelderin hat überraschenderweise herausgefunden, daß durch die Kombination von Okoubaka und 3,3',4',5,7-Pentahydroxyflavon zusammen mit mindestens einer Galactosidase (erfindungsgemäß Lactase) und mit Pyridoxinhydrochlorid (Vitamin B6) die zuvor geschilderte Aufgabenstellung in effizienter Weise gelöst und ein wirksames Therapeutikum für die Behandlung von Nahrungsmittelunverträglichkeiten der eingangs genannten Art, insbesondere Nahrungsmittelunverträglichkeiten mit Magen/Darm-Beschwerden, bereitgestellt werden kann, welches vollkommen nebenwirkungsfrei ist.

Okoubaka und 3,3',4',5,7-Pentahydroxyflavon stärken beide die Dannschleimhaut gegen unverträgliche Stoffe. Insbesondere wirkt Okoubaka aus der Rinde des im westafrikanischen Regenwald beheimateten Baumes Okoubaka aubrevillei den typischen, bei Nahrungsmittelunverträglichkeiten auftretenden Magen/Darm-Beschwerden entgegen. Die Inhaltsstoffe von Okoubaka, insbesondere in der Kombination mit 3,3',4',5,7-Pentahydroxyflavon, vorzugsweise pflanzlichem 3,3',4',5,7-Pentahydroxyflavon, führen dazu, daß die Darmschleimhaut über vielfältige physikalische Effekte stabilisiert wird und so weniger empfindlich auf unverträgliche Substanzen reagiert. Das 3,3',4',5,7-Pentahydroxyflavon wirkt als Adstringens und entfaltet auf diese Weise adstringierende Wirkung auf die Darmoberfläche bzw. Darmschleimhaut und macht diese weniger empfindlich in bezug auf die unverträglichen Substanzen.

Die Lactase unterstützt dieses Geschehen, indem der mit der Nahrung aufgenommene Milchzucker (Lactose) sofort gespalten wird. Die für eine Milchzuckerunverträglichkeit typischen Symptome können dadurch ebenfalls gelindert werden.

Im Rahmen der erfindungsgemäßen Zusammensetzung trägt das Pyridoxinhydrochlorid (Vitamin B6) gleichermaßen zur Stärkung der Darmschleimhaut bei und unterstützt und verstärkt somit die diesbezügliche Wirkung von Okoubaka und 3,3',4',5,7-Pentahydroxyflavon.

Durch die erfindungsgemäß vorgesehene Wirkstoffkombination aus Okoubaka und 3,3',4',5,7-Pentahydroxyflavon zusammen mit Lactase und mit Pyridoxinhydrochlorid (Vitamin B6) werden durch physikalische Prozesse im Magen/Darm-Trakt die typischen Symptome von Nahrungsmittelunverträglichkeiten, wie insbesondere Bauchschmerzen, Übelkeit, Blähungen und Durchfall, in effizienter Weise gemindert bzw. verhindert.

Die erfindungsgemäße Kombination wirkt den typischen Magen/Darm-Beschwerden, wie sie bei Nahrungsmittelunverträglichkeiten auftreten können, entgegen, Aufgrund ihrer unter anderem adstringierenden Wirkung, kombiniert mit einer pH-Verschiebung, führt die erfindungsgemäße Zusammensetzung sozusagen zu einem Abdichten der Darmwand und somit zu einer reduzierten Empfindlichkeit gegenüber unverträglichen Substanzen. Durch die Inhaltsstoffe der erfindungsgemäßen Zusammensetzung wird also der pH-Wert im Darm verändert, damit die körpereigenen Stoffe wieder eine entsprechende Umgebung haben, um zu wirken. Diese Wirkungen werden vor allem durch die biologischen Inhaltsstoffe herbeigeführt.

Wie zuvor beschrieben, wird der zuvor eingesetzte Wirkstoff Okoubaka aus der Rinde des im westafrikanischen Regenwald beheimateten Okoubaka aubrevillei Baumes gewonnen. Üblicherweise wird Okoubaka in Form eines Extraktes (z. B. wäßriger, wäßrig-alkoholischer oder alkoholischer Extrakt) eingesetzt. Erfindungsgemäß ist es vorgesehen, daß Okoubaka in homöopathischer Form bzw. Menge von Dil. D4 eingesetzt wird. Alle vorstehenden und nachfolgenden Mengen- und Gewichtsprozentangaben sind so zu verstehen, daß sie sich auf die Extraktform bzw. die homöopathische Form beziehen (d.h. die erfindungsgemäße Zusammensetzung 12 bis 18 Gew.-% Okoubaka Dil. D4 enthält).

Was das erfindungsgemäß eingesetzte 3,3',4',5,7-Pentahydroxyflavon anbelangt, so kommt erfindungsgemäß bevorzugt pflanzliches 3,3',4',5,7-Pentahydroxyflavon zum Einsatz. 3,3',4',5,7-Pentahydroxyflavon entspricht der chemischen Summenformel C₁₅H₁₀O₇ mit einer relativen Molmasse von 302,23. 3,3',4',5,7-Pentahydroxyflavon wird synonym auch als Quercetin bezeichnet. Als Glykosid ist 3,3',4',5,7-Pentahydroxyflavon sehr weit verbreitet in Baumrinden, insbesondere der amerikanischen Färbereiche und anderer Eichenarten sowie der Douglasfichte, ferner in Rinden und Schalen vieler Früchte und Gemüse sowie in deren Blättern, auch in gelben Blüten.

Was die erfindungsgemäß eingesetzte Galactosidase anbelangt, so wird erfindungsgemäß als Galactosidase Lactase (β-D-Galactosid-Galactohydrolase) eingesetzt.

Was das erfindungsgemäß eingesetzte Pyridoxin anbelangt, so wird dieses in Form von Pyridoxinhydrochlorid (Vitamin B6) eingesetzt. Wie zuvor beschrieben, trägt im Rahmen der erfindungsgemäßen Zusammensetzung das Pyridoxin in Form von Pyridoxinhydrochlorid (Vitamin B6) gleichermaßen zur Stärkung der Darmschleimhaut bei.

Neben den vorgenannten Wirk- bzw. Inhaltsstoffen enthält die erfindungsgemäße Zusammensetzung außerdem mindestens einen pharmazeutischen Träger bzw. Exzipienten.

Erfindungsgemäß liegt die erfindungsgemäße Zusammensetzung in peroral verabreichbarer Applikationsform vor. Dabei liegt die erfindungsgemäße Zusammensetzung in fester Dosierungsform vor. Die feste Dosierungsform ist als Filmtablette oder Kapsel mit einer magensaftresistenten und/oder dünndarmlöslichen Umhüllung ausgebildet. Auf diese Weise entfaltet die erfindungsgemäße Zusammensetzung am gewünschten Ort, nämlich im Darm, die angestrebte Wirkung.

Wie zuvor beschrieben, enthält die erfindungsgemäße Zusammensetzung eine Kombination der Wirkstoffe (a) Okoubaka, (b) 3,3',4',5,7-Pentahydroxyflavon, (c) Galactosidase(n) und (d) Pyridoxin, insbesondere Pyridoxinhydrochlorid (Vitamin B6).

Die Menge an den jeweiligen Wirk- bzw. Inhaltsstoffen in der erfindungsgemäßen Zusammensetzung kann in weiten Bereichen variieren. Es versteht sich von selbst, daß bei den Mengenangaben im Fall von Prozentangaben, insbesondere Gewichtsprozentangaben, die Inhaltsstoffe in ihrer Gesamtheit so zu kombinieren sind, daß die Prozentangaben insgesamt stets 100 % ergeben. Im übrigen gilt, daß der Fachmann anwendungsbezogen oder einzelfallbedingt von den nachfolgend aufgeführten Mengenangaben abweichen kann, ohne daß er den Rahmen der vorliegenden Erfindung verläßt.

Was die Menge an dem Wirkstoff Okoubaka, anbelangt, so enthält die erfindungsgemäße Zusammensetzung Okoubaka, in Form von Dil. D4 in Mengen von 12 bis 18 Gew.-%, bezogen auf die Zusammensetzung. Die vorstehenden Gewichtsprozentangaben sind so zu verstehen, daß sie sich auf die Extraktform bzw, die homöopathische Form beziehen.

Erfindungsgemäß enthält die erfindungsgemäße Zusammensetzung 3,3',4',5,7-Pentahydroxyflavon in Mengen von 5 bis 10 Gew.-%, bezogen auf die Zusammensetzung.

Was die Menge an Galactosidase(n) in der erfindungsgemäßen Zusammensetzung anbelangt, so enthält die erfindungsgemäße Zusammensetzung Galactosidase(n) in Mengen von 0,5 bis 5 Gew.-%, bezogen auf die Zusammensetzung.

Was die Menge an Pyridoxinhydrochlorid (Vitamin B6) in der erfindungsgemäßen Zusammensetzung anbelangt, so enthält die erfindungsgemäße Zusammensetzung Pyridoxinhydrochlorid (Vitamin B6) in Mengen von 0,1 bis 1 Ges.-%, bezogen auf die Zusammensetzung.

Neben den relativen bzw. prozentualen Mengenanteilen der Wirk- bzw. Inhaltsstoffe in der erfindungsgemäßen Zusammensetzung spielt auch die absolute Konzentration bzw. die absolute Menge pro Desiereinheit, insbesondere je Tablette, eine entscheidende Rolle in bezug auf die Wirksamkeit der erfindungsgemäßen Zusammensetzung:
Im allgemeinen enthält die erfindungsgemäße pharmazeutische Zusammensetzung den Wirkstoff Okoubaka in Form von Dil. D4 pro Dosiereinheit, insbesondere je Tablette, in Mengen von 1 bis 1.000 mg, insbesondere 10 bis 500 mg, vorzugsweise 50 bis 250 mg, besonders bevorzugt 75 bis 200 mg, ganz besonders bevorzugt 80 bis 150 mg. Die vorstehenden Mengenangaben sind so zu verstehen, daß sie sich auf die Extraktform bzw. die homöopathische Form beziehen.

Was das 3,3',4',5,7-Pentahydroxyflavon anbelangt, so enthält die erfindungsgemäße Zusammensetzung das 3,3',4',5,7-Pentahydroxyflavon, insbesondere in pflanzlicher Form, pro Dosiereinheit, insbesondere je Tablette, in Mengen von 10 bis 500 mg, insbesondere 20 bis 300 mg, vorzugsweise 30 bis 200 mg, besonders bevorzugt 40 bis 100 mg, ganz besonders bevorzugt 45 bis 80 mg. Dennoch kann es im Einzelfall oder anwendungsbezogen erforderlich sein, von den vorgenannten Mengenangaben abzuweichen, ohne daß der Fachmann den Rahmen der vorliegenden Erfindung verläßt.

Was die Menge der eingesetzten Galactosidase(n) pro Dosiereinheit anbelangt, so kann die Zusammensetzung Galactosidase(n) pro Dosiereinheit, insbesondere je Tablette, in Mengen von 1 bis 200 mg, insbesondere 2 bis 150 mg, vorzugsweise 5 bis 100 mg, besonders bevorzugt 10 bis 50 mg, ganz besonders bevorzugt 15 bis 30 mg, enthalten. Dennoch kann es im Einzelfall oder anwendungsbezogen erforderlich sein, von den vorgenannten Mengenangaben abzuweichen, ohne daß der Fachmann den Rahmen der vorliegenden Erfindung verläßt.

Was die Menge des eingesetzten Pyridoxinhydrochlorids (Vitamin B6) pro Dosiereinheit anbelangt, so kann die Zusammensetzung Pyridoxin, insbesondere Pyridoxinhydrochlorid (Vitamin B6), pro Dosiereinheit, insbesondere je Tablette, in Mengen von 0,01 bis 10 mg, insbesondere 0,05 bis 5 mg, vorzugsweise 0,1 bis 2,5 mg, besonders bevorzugt 0,5 bis 2 mg, ganz besonders bevorzugt 1 bis 2 mg, enthalten. Dennoch kann es im Einzelfall oder anwendungsbezogen erforderlich sein, von den vorgenannten Mengenangaben abzuweichen, ohne daß der Fachmann den Rahmen der vorliegenden Erfindung verläßt.

Neben den vorgenannten Wirk- bzw. Inhaltsstoffen enthält die erfindungsgemäße Zusammensetzung außerdem Ascorbinsäure (Vitamin C). Die Ascorbinsäure unterstützt im Rahmen der erfindungsgemäßen Zusammensetzung die effiziente Wirkweise der anderen Wirk- bzw. Inhaltsstoffe und hilft insbesondere, unerwünschte Oxidationseffekte zu vermeiden, und stabilisiert auf diese Weise zusätzlich die Darmschleimhaut.

Erfindungsgemäß wird die Ascorbinsäure in Form einer natürlichen Ascorbinsäurequelle zugesetzt, nämlich in Form von Acerolapulver.

Acerola (*Malpighia glabra,* ehemals auch *Malpighia punicxfolia),* auch Acerolakirsche, Ahornkirsche, Antillenkirsche, Kirsche der Antillen, Puerto Rico Kirsche oder Westindische Kirsche genannt, gehört botanisch zu der Familie der Malpighiengewächse (*Malpighiaceae*), zu denen auch die Lianen gehören. Die Acerolakirsche ist zwar wie die heimische Kirsche eine Steinfrucht, jedoch botanisch nicht mit dieser verwandt, da letztere zu der Familie der Rosengewächse gehört. Die Acerolakirsche ist - neben Camu-Camu (*Myrciaria dubia*) *-* die ascorbinsäurereichste aller bekannten Früchte (bis zu 3.000 mg pro 100 g Fruchtfleisch); außerdem enthält die Acerolakirsche Vitamin B6, Provitamin A sowie Eisen, Calcium, Magnesium, Thiamin, Riboflavin und Niacin.

Die erfindungsgemäße Zusammensetzung enthält Acerolapulver in Mengen von 20 bis 30 Gew.-%, bezogen auf die Zusammensetzung. In absoluter Menge kann die erfindungsgemäße Zusammensetzung das Acerolapulver pro Dosiereinheit, insbesondere je Tablette, in Mengen von 10 bis 400 mg, insbesondere 50 bis 300 mg, vorzugsweise 75 bis 250 mg, besonders bevorzugt 100 bis 200 mg, ganz besonders bevorzugt 125 bis 200 mg, enthalten. Dennoch kann es im Einzelfall oder anwendungsbezogen erforderlich sein, von den vorgenannten Mengenangaben abzuweichen, ohne daß der Fachmann den Rahmen der vorliegenden Erfindung verläßt.

Gemäß einer besonderen Ausführungsform der vorliegenden Erfindung betrifft die vorliegende Erfindung eine Zusammensetzung in Form einer pharmazeutischen Zubereitung, welche sich für die prophylaktische und/oder kurative Behandlung von Nahrungsmittelunverträglichkeiten mit Magen/Darm-Beschwerden eignet, wobei die Zusammensetzung - in Kombination und in jeweils wirksamen, insbesondere pharmazeutisch wirksamen Mengen - enthält:
(a) Okoubaka, wobei die Zusammensetzung Okoubaka in Form von Dil. D4 pro Dosiereinheit, insbesondere je Tablette, in Mengen von 1 bis 1.000 mg, insbesondere 10 bis 500 mg, vorzugsweise 50 bis 250 mg, besonders bevorzugt 75 bis 200 mg, ganz besonders bevorzugt 80 bis 150 mg, enthält;
(b) 3,3',4',5,7-Pentahydroxyflavon, wobei die Zusammensetzung 3,3',4',5,7-Pentahydroxyflavon pro Dosiereinheit, insbesondere je Tablette, in Mengen von 10 bis 500 mg, insbesondere 20 bis 300 mg, vorzugsweise 30 bis 200 mg, besonders bevorzugt 40 bis 100 mg, ganz besonders bevorzugt 45 bis 80 mg, enthält;
(c) β-D-Galactosid-Galactohydrolase (Lactase), wobei die Zusammensetzung β-D-Galactosid-Galactohydrolase (Lactase) pro Dosiereinheit, insbesondere je Tablette, in Mengen von 1 bis 200 mg, insbesondere 2 bis 150 mg, vorzugsweise 5 bis 100 mg, besonders bevorzugt 10 bis 50 mg, ganz besonders bevorzugt 15 bis 30 mg, enthält;
(d) Pyridoxinhydrochlorid (Vitamin B6), wobei die Zusammensetzung Pyridoxinhydrochlorid (Vitamin B6) pro Dosiereinheit, insbesondere je Tablette, in Mengen von 0,01 bis 10 mg, insbesondere 0,05 bis 5 mg, vorzugsweise 0,1 bis 2,5 mg, besonders bevorzugt 0,5 bis 2 mg, ganz besonders bevorzugt 1 bis 2 mg, enthält; und
(e) Ascorbinsäure (Vitamin C) in Form von Acerolapulver, wobei die Zusammensetzung Acerolapulver pro Dosiereinheit, insbesondere je Tablette, in Mengen von 10 bis 400 mg, insbesondere 50 bis 300 mg, vorzugsweise 75 bis 250 mg, besonders bevorzugt 100 bis 200 mg, ganz besonders bevorzugt 125 bis 200 mg, enthält.

In bezug auf die zuvor geschilderten besonderen Ausführungsformen der vorliegenden Erfindung gelten die vorstehenden Ausführungen zu den allgemeinen Ausführungsformen entsprechen,

Neben den vorgenannten Wirk- und Inhaltsstoffen kann die erfindungsgemäße Zusammensetzung außerdem weitere Inhaltsstoffe enthalten. Diese können insbesondere ausgewählt sein aus Zusatz- und/oder Hilfsstoffen, wie Füll-, Streck-, Binde-, Netz-, Stabilisierungs-, Färbe-, Puffer-, Riech-, Geschmacks-, Süßungs-, Aroma-, Verarbeitungshilfs- und/oder Konservierungsstoffen und -mitteln.

Die erfindungsgemäße Zusammensetzung kann insbesondere als Arzneimittel oder Pharmazeutikum, als Medizinprodukt, als Homöopathikum oder aber als Nahrungsergänzungsmittel, Nahrungsmittelzusatz oder Diätetikum ausgebildet sein.

Erfindungsgemäß liegt die erfindungsgemäße pharmazeutische Zusammensetzung bzw. Zubereitung, wie sie vorstehend beschrieben ist, in Dosiereinheiten in Form von Kapseln oder Filmtabletten, bevorzugt Filmtabletten, in Dosiereinheiten von 0,1 bis 5 g, insbesondere 0,25 bis 2,5 g, vorzugsweise 0,4 bis 1,5 g, vor. Diese Dosiereinheiten sind vorteilhafterweise vor einer Mahlzeit, insbesondere 1 bis 60 Minuten vor einer Mahlzeit, vorzugsweise 5 bis 15 Minuten vor einer Mahlzeit, peroral zur applizieren, und zwar jeweils in Mengen von 1 bis 2 Dosiereinheiten, und zwar in Abhängigkeit von der Schwere des Krankheitsbildes.

Die zuvor beschriebene Zusammensetzung ist ein sicheres Therapeutikum zur prophylaktischen und/oder kurativen, insbesondere symptomatischen Behandlung von Nahrungsmittelunverträglichkeiten, insbesondere Nahrungsmittelunverträglichkeiten mit Magen/Darm-Beschwerden, der eingangs genannten Art. Die erfindungsgemäße Zusammensetzung führt zu keinen bzw. nahezu keinen systemischen Nebenwirkungen, gewährleistet aber dennoch eine sichere symptomatische Therapie dieser Erkrankungen.

Des weiteren betrifft die vorliegende Erfindung - gemäß einem **zweiten** Aspekt der vorliegenden Erfindung - die Verwendung der erfindungsgemäßen Zusammensetzung zur Herstellung eines Arzneimittels zur prophylaktischen und/oder kurativen, insbesondere symptomatischen prophylaktischen und/oder kurativen Behandlung von Nahrungsmittelunverträglichkeiten mit Magen/Darm-Beschwerden.

Insbesondere betrifft die vorliegende Erfindung die Verwendung der zuvor beschriebenen erfindungsgemäßen Zusammensetzung zur prophylaktischen und/oder kurativen, insbesondere symptomatischen prophylaktischen und/oder kurativen Behandlung bzw. zur Herstellung eines Arzneimittels zur prophylaktischen und/oder kurative, insbesondere symptomatischen prophylaktischen und/oder kurativen Behandlung von Kohlenhydratmalabsorption, insbesondere Monosaccharidmalabsorption und Disaccharidmalabsorption, wie Laktoseintoleranz (Laktoseunverträglichkeit, Alaktasie), Saccharoseintoleranz und Maltosintoleranz.

Bei der erfindungsgemäßen Verwendung wird die erfindungsgemäße Zusammensetzung üblicherweise vor einer Mahlzeit, insbesondere 1 bis 60 Minuten vor Aufnahme des Essens, vorzugsweise 5 bis 15 Minuten vor Aufnahme des Essens, peroral eingenommen bzw. appliziert. Diesbezüglich kann auf die vorstehenden Ausführungen verwiesen werden.

Weitere Ausgestaltungen, Abwandlungen und Variationen sowie Vorteile der vorliegenden Erfindung sind für den Fachmann beim Lesen der Beschreibung ohne weiteres erkennbar und realisierbar, ohne daß er dabei den Rahmen der vorliegenden Erfindung verläßt.

Das nachfolgende Ausführungsbeispiel dient lediglich der Veranschaulichung der vorliegenden Erfindung, ohne sie jedoch hierauf zu beschränken.

### Ausführungsbeispiel:

Es wurden Filmtabletten auf Basis einer erfindungsgemäßen Wirkstoffkombination von Okoubaka (Dil. D4), Ascorbinsäure (Acerolapulver), 3,3',4',5,7-Pentahydroxyflavon, Lactase und Pyridoxinhydrochlorid (Vitamin B6) hergestellt, wobei die hergestellten Filmtabletten enthielten:
- Okoubaka Dil. D4 100,000 mg
- Acerolapulver 166,660 mg
   (entspricht ca. 33 mg Vitamin C)
- Lactase 23,000 mg
- 3,3',4',5,7-Pentahydroxyflavon (Quercetin)
   (pflanzlich) 50,000 mg
- Pyridoxinhydrochlorid (Vitamin B6) 1,336 mg

Die Filmtabletten hatten einen magensaftresistenten, dünndarmlöslichen Überzug.

Die vorstehenden Filmtabletten wurden an 15 Probanden mit Lactoseintoleranz (Milchzuckerunverträglichkeit) über 10 Tage getestet. Zur Sicherung der Diagnose wurde ein oraler Milchzuckerbelastungstest mit 50 g Milchzucker durchgeführt, wobei bei allen Probanden die Lactoseintoleranz sowohl über den Anstieg des Wasserstoffgehaltes in der Atemluft als auch durch den fehlenden bzw. zu geringen Blutzuckeranstieg nachgewiesen wurde (Wasserstoffanstieg jeweils > 20 ppm; Blutzuckeranstieg jeweils < 20 mg/dl). Alle Probanden litten an physiologischem, erworbenem Lactasemangel und klagten im Fall des Verzehrs von Milch und Käseprodukten über Bauchschmerzen, Völlegefühl, Blähungen, Übelkeit und Durchfall.

Allen Probanden wurde zu den Hauptznahlzeiten (Frühstück, Mittagessen, Abendessen) Nahrung mit mäßigem Lactoseanteil (Lactosemenge <10g/ diem verabreicht). An den ersten zwei der zehn Teststage wurde den Probanden kein Therapeutikum vor den Mahlzeiten verabreicht. Alle Probanden klagten über Übelkeit, Völlegefühl und Blähungen, 10 der Probanden auch über leichten Durchfall.

Ab dem dritten Tag bis zum zehnten Tag wurden den Probanden 10 Minuten vor den Mahlzeiten jeweils zwei der zuvor beschriebenen Tabletten verabreicht. Bei 11 der 15 Probanden verschwanden daraufhin die Beschwerden vollständig. Bei den übrigen 4 Probanden gingen diese Beschwerden merklich zurück.

Bei jeweils weiteren 5 Probanden wurden vier Vergleichszusammensetzungen in analoger Weise, wie zuvor beschrieben, getestet. Die Vergleichszusammensetzungen unterschieden sich von der zuvor definierten erfindungsgemäßen Zusammensetzung dadurch, daß die erste Vergleichszusammensetzung ohne Okoubaka formuliert war, die zweite Vergleichszusammensetzung ohne Lactase formuliert war, die dritte Vergleichszusammensetzung ohne 3,3',4',5,7-Pentahydroxyflavon (Quercetin) formuliert war und die vierte Vergleichszusammensetzung ohne Pyridoxinhydrochlorid (Vitamin B6) formuliert war. Bei allen vier Vergleichszusammensetzungen litten die Probanden nach wie vor unter den zuvor geschilderten Beschwerden.

Die vorstehenden Versuche belegen eindrucksvoll die Wirksamkeit der erfindungsgemäßen Zusammensetzung bei der Behandlung von Nahrungsmittelunverträglichkeiten. Nur im Fall der erfindungsgemäßen Zusammensetzung auf Basis einer quarternären Kombination von Okoubaka, 3,3',4',5,7-Pentahydroxyflavon (Quercetin), Lactase und Pyridoxinhydrochlorid (Vitamin B6) wird der gewünschte Wirkeffekt beobachtet.

## Patentansprüche

1. Zusammensetzung in Form einer pharmazeutischen Zubereitung zur Anwendung bei der prophylaktischen und/oder kurativen Behandlung von Nahrungsmittelunverträglichkeiten mit Magen/Darm-Beschwerden, wobei die Zusammensetzung - in Kombination und in jeweils pharmazeutisch wirksamen Mengen - enthält:
(a) Okoubaka, wobei die Zusammensetzung Okoubaka in Form eines Extraktes in homöopathischer Form und Menge von Dil. D4 in Mengen von 12 bis 18 Gew.-%, bezogen auf die Zusammensetzung, enthält;
(b) 3,3',4',5,7-Pentahydroxyflavon, wobei die Zusammensetzung 3,3',4',5,7-Pentahydroxyflavon in Mengen von 5 bis 10 Gew.-%, bezogen auf die Zusammensetzung, enthält;
(c) β-D-Galactosid-Galactohydrolase (Lactase), wobei die Zusammensetzung β-D-Galactosid-Galactohydrolase (Lactase) in Mengen von 0,5 bis 5 Gew.-%, bezogen auf die Zusammensetzung, enthält;
(d) Pyridoxinhydrochlorid (Vitamin B6), wobei die Zusammensetzung Pyridoxinhydrochlorid (Vitamin B6) in Mengen von 0,1 bis 1 Gew.-%, bezogen auf die Zusammensetzung, enthält; und
(e) Ascorbinsäure (Vitamin C) in Form von Acerolapulver, wobei die Zusammensetzung Acerolapulver in Mengen von 20 bis 30 Gew.-%, bezogen auf die Zusammensetzung, enthält;
wobei die Zusammensetzung außerdem einen pharmazeutischen Träger enthält und
wobei die Zusammensetzung in peroral verabreichbarer Applikationsform in fester Dosierungsform als Filmtablette oder Kapsel mit magensaftresistentem und/oder dünndarmlöslichem Überzug oder Umhüllung vorliegt.

2. Zusammensetzung zur Anwendung nach Anspruch 1, wobei die Zusammensetzung Okoubaka in Form eines wäßrigen, alkoholischen oder wäßrig-alkoholischen Extraktes enthält.

3. Zusammensetzung zur Anwendung nach Anspruch 1 oder 2, wobei Okoubaka aus der Rinde von Okoubaka aubrevillei gewonnen ist.

4. Verwendung einer Zusammensetzung nach den vorangehenden Ansprüchen zur Herstellung eines Arzneimittels zur prophylaktischen und/oder kurativen, insbesondere symptomatischen prophylaktischen und/oder kurativen Behandlung von Nahrungsmittelunverträglichkeiten, insbesondere Nahrungsmittelunverträglichkeiten mit Magen/Darm-Beschwerden.

5. Verwendung einer Zusammensetzung nach den vorangehenden Ansprüchen zur Herstellung eines Arzneimittels zur prophylaktischen und/oder kurativen, insbesondere symptomatischen prophylaktischen und/oder kurativen Behandlung von Kohlenhydratmalabsorption, insbesondere Monosaccharidmalabsorption und Disaccharidmalabsorption, wie Laktoseintoleranz (Laktoseunverträglichkeit, Alaktasie), Saccharoseintoleranz und Maltosintoleranz.

## Claims

1. Composition in the form of a pharmaceutical formulation for use in the prophylactic and/or curative treatment of food intolerances with stomach/intestinal disorders, wherein the composition - in combination with respectively pharmaceutically effective amounts - contains:
(a) Okoubaka, wherein the Okoubaka composition contains an extract in homeopathic form and amount of Dil. D4 in amounts of 12 to 18 wt.-%, based on the composition;
(b) 3,3',4',5,7-pentahydroxyflavone, wherein the composition contains 3,3',4',5,7-pentahydroxyflavone in amounts of from 5 to 10 wt.-%, based on the composition;
(c) β-D-galactoside-galactohydrolase (lactase), wherein the composition contains β-D-galactoside-galactohydrolase (lactase) in amounts of from 0.5 to 5 wt.-%, based on the composition;
(d) pyridoxine hydrochloride (vitamin B6), wherein the composition contains pyridoxine hydrochloride (vitamin B6) in amounts of from 0.1 to 1 wt.-%, based on the composition; and
(e) ascorbic acid (vitamin C) in the form of acerola powder, wherein the composition contains acerola powder in amounts of 20 to 30 wt.-%, based on the composition;
wherein the composition further contains a pharmaceutical carrier, and
wherein the composition is in orally administrable administration form in solid dosage form as a film tablet or capsule with an enteric and/or small intestinal dissoluble coating or wrapping.

2. Composition for use according to claim 1,
wherein the composition contains Okoubaka in the form of an aqueous, alcoholic or aqueous-alcoholic extract.

3. Composition for use according to claim 1 or 2,
wherein Okoubaka is extracted from the bark of Okoubaka aubrevillei.

4. Use of a composition according to the preceding claims for producing a medicament for the prophylactic and/or curative, particularly symptomatic prophylactic and/or curative treatment of food allergies, especially food allergies with stomach/intestinal disorders.

5. Use of a composition according to the preceding claims for preparing a medicament for prophylactic and/or curative, particularly symptomatic prophylactic and/or curative treatment of carbohydrate malabsorption, in particular monosaccharide malabsorption and disaccharide malabsorption such as lactose intolerance (lactose incompatibility, alactasia), sucrose intolerance and maltose intolerance.

## Revendications

1. Composition sous forme d'une préparation pharmaceutique pour une utilisation lors du traitement prophylactique et/ou curatif d'intolérances alimentaires avec troubles gastrointestinaux, la composition contenant - en association, et chacun en des quantités pharmaceutiquement efficaces - :
(a) de l'okoubaka, la composition contenant l'okoubaka sous forme d'un extrait sous forme homéopathique et en une quantité de Dil.D4, en des quantités de 12 à 18 % en poids par rapport à la composition ;
(b) de la 3,3',4',5,7-pentahydroxyflavone, la composition contenant la 3,3',4',5,7-pentahydroxyflavone en des quantités de 5 à 10 % en poids par rapport à la composition ;
(c) de la β-D-galactoside-galactohydrolase (lactase), la composition contenant la β-D-galactoside-galactohydrolase (lactase) en des quantités de 0,5 à 5 % en poids par rapport à la composition ;
(d) du chlorhydrate de pyridoxine (vitamine B6), la composition contenant le chlorhydrate de pyridoxine (vitamine B6) en des quantités de 0,1 à 1 % en poids par rapport à la composition ; et
(e) de l'acide ascorbique (vitamine C) sous forme de poudre d'acérola, la composition contenant la poudre d'acérola en des quantités de 20 à 30 % en poids par rapport à la composition,
la composition contenant par ailleurs du support pharmaceutique, et
la composition se présentant sous une forme galénique pouvant être administrée par voie orale, sous forme posologique solide, sous forme d'un comprimé enrobé ou d'une gélule comportant un enrobage ou un revêtement gastro-résistant et/ou entéro-soluble.

2. Composition pour l'utilisation selon la revendication 1, la composition contenant de l'okoubaka sous forme d'un extrait aqueux, alcoolique ou hydro-alcoolique.

3. Composition pour l'utilisation selon la revendication 1 ou 2, dans laquelle l'okoubaka a été obtenu à partir de l'écorce d'Okoubaka aubrevillei.

4. Utilisation d'une composition selon les revendications précédentes pour fabriquer un médicament destiné au traitement prophylactique et/ou curatif, en particulier symptomatique, prophylactique et/ou curatif, d'intolérances alimentaires, en particulier d'intolérances alimentaires avec troubles gastro-intestinaux.

5. Utilisation d'une composition selon les revendications précédentes pour fabriquer un médicament destiné au traitement prophylactique et/ou curatif, en particulier symptomatique, prophylactique et/ou curatif, de la malabsorption des glucides, en particulier de la malabsorption des monosaccharides et de la malabsorption des disaccharides, telles que l'intolérance au lactose (intolérance au lactose, alactasie), l'intolérance au saccharose et l'intolérance au maltose.
